# EUROPEAN PATENT SPECIFICATION

(11) **EP 2 794 026 B1**
(45) Date of publication and mention of the grant of the patent: **28.10.2015**
(21) Application number: 12791481.0
(22) Date of filing: 26.11.2012
(51) Int. Cl.: A61Q 5/12, A61K 8/02, A61K 8/34, A61K 8/41, A61K 8/42, A61K 8/898

(54) **METHOD FOR PRODUCTION OF STRUCTURED LIQUID AND STRUCTURED LIQUID**
VERFAHREN ZUR HERSTELLUNG EINER STRUKTURIERTEN FLÜSSIGKEIT UND STRUKTURIERTE FLÜSSIGKEIT
PROCÉDÉ DE PRÉPARATION D'UN LIQUIDE STRUCTURÉ ET LIQUIDE STRUCTURÉ

(30) Priority: 20.12.2011 EP 11194652
(43) Date of publication of application: 29.10.2014
(73) Proprietor: Unilever N.V., 3013 AL Rotterdam (NL); Unilever PLC, London, Greater London EC4Y 0DY (GB)
(72) Inventor: EGAN, Michael, John, Liverpool Merseyside L69 7ZD (GB); IRVING, Graeme, Neil, Wirral Merseyside CH63 3JW (GB)
(74) Representative: Fijnvandraat, Arnoldus
(86) International application number: PCT/EP2012/073616
(87) International publication number: WO 2013/092118

(56) References cited:
- WO-A1-2008/064976
- WO-A1-2010/089320
- US-A1- 2005 233 935
- US-A1- 2006 040 837
- US-B1- 6 345 907
- US-B2- 6 534 457

## Description

### FIELD OF THE INVENTION

The present invention relates to a method for the production of a structured liquid that can be used as hair conditioner, by using a Controlled Deformation Dynamic Mixer. The present invention also relates to a structured liquid containing fatty compound, cationic surfactant, and water, that has a high viscosity and obtained by the present method.

### BACKGROUND TO THE INVENTION

Mixing can be described as either distributive or dispersive. In a multi-phase material comprising discrete domains of each phase, distributive mixing seeks to change the relative spatial positions of the domains of each phase, whereas dispersive mixing seeks to overcome cohesive forces to alter the size and size distribution of the domains of each phase. Most mixers employ a combination of distributive and dispersive mixing although, depending on the intended application the balance will alter. For example a machine for mixing peanuts and raisins will be wholly distributive so as not to damage the things being mixed, whereas a blender/homogeniser will be dispersive.

### Mixing Devices

EP 194 812 A2 discloses a cavity transfer mixer (CTM). Also WO 96/20270 describes a 'Cavity Transfer Mixer', comprising confronting surfaces, each having a series of cavities formed therein in which the surfaces move relatively to each other and in which a liquid material is passed between the surfaces and flows along a pathway successively passing through the cavities in each surface. Generally, the cross-sectional area for flow varies by a factor of less than 3 through the apparatus.

WO 96/20270 also describes a mixer, hereinafter referred to as a 'Controlled Deformation Dynamic Mixer' (CDDM). In common with the CTM, this type of mixer has stator and rotor elements with opposed cavities which, as the mixer operates, move past each other across the direction of bulk flow through the mixer. It is distinguished from the CTM in that material is also subjected to extensional deformation. The extensional flow and efficient dispersive mixing is secured by having confronting surfaces with cavities arranged such that the cross sectional area for bulk flow of the liquid through the mixer successively increases and decreases by a factor of at least 5 through the apparatus. The CDDM combines the distributive mixing performance of the CTM with dispersive mixing performance.

WO 96/20270 further describes that this type of mixer can be used for the production of structured liquids, such as compositions containing surfactants (anionic, cationic, non-ionic, zwitterionic). The production of a fabric conditioning composition is described, wherein first distearyldimethyl ammonium chloride (80%) and water (20%) are mixed to a liquid crystal composition, while heating. Then water is added, to produce a homogeneous saturated liquid crystal mixture with 30% active and 70% water, also under heating. This mixture is fed to the CDDM with more water (at ambient temperature), leading to inversion of the liquid crystal phase, and a homogeneous dispersion contain between 5 and 12% active. The viscosity of the produced compositions ranges from 40 to 155 mPa.s. Advantage of the process is that not only a homogeneous dispersion is obtained, but also that not all water in the composition needs to be heated (as is the case in the conventional mixing process).

WO 2010/089320 A1, WO 2010/089322 A1, and WO 2010/091983 A1 disclose specific types of a distributive and dispersive mixing apparatus of the CDDM type or CTM type, comprising two confronting surfaces having cavities therein.

### Hair Conditioners

Hair conditioners are used by consumers to treat their hair, generally after washing it. Hair conditioners may enhance the appearance and texture of the hair and facilitates styling. Conditioning agents will also improve gloss, sheen and texture of damaged hair. Typical ingredients of hair conditioners are water, viscosifier (e.g. cetearyl alcohol emulsifying wax, may also act as moisturiser), hair conditioning agents (such as e.g. dimethiconol (a silicone polymer), stearamidopropyl dimethylamine (a lipid based amine compound), behentrimonium chloride (a cationic surfactant), glycerin, solvent (e.g. dipropylene glycol), preservatives, sequestrant (e.g. disodium EDTA), anionic surfactant (e.g. TEA-dodecylbenzenesulfonate, sodium cetylstearyl sulphate), moisturiser (humectants such as glycerol, and propylene glycol), fragrances, and colourants.

Hair conditioner is an example of a structured liquid, of which its sensory and functional properties are governed by its microstructure in addition to the ingredients of the product. Surfactants in solutions generally form micelles. At sufficiently high concentrations, micelles in solution will interact and a phase change to liquid crystal dispersions will occur. So as surfactant concentration increases an isotropic micellar solution is formed. Further increases may yield a discontinuous dispersion of liquid crystals in cubic and hexagonal arrangements. At even higher concentrations lamellar dispersions are likely to form. At some point the system inverts and becomes a dispersion of water in surfactant liquid crystals.

The viscosity of such products is a function of phase and the high viscosity cubic and hexagonal phases typically appear as gels that generally cannot be mixed using conventional equipment. A micelle solution generally has a low viscosity, while lamellar liquid crystal dispersions has a high viscosity and exhibits shear thinning. Cubic and hexagonal liquid crystal dispersions are generally highly viscous. The physical properties of micellar solutions and liquid crystal dispersions vary significantly.

The hair conditioners are usually produced by either of two processes. The first process is melting a fatty compound like fatty alcohol, and mixing it with the cationic surfactant in order to make an oil phase. This oil phase is then mixed with the aqueous phase. This is usually done at temperatures above the melting temperature of the fatty compound, typically at a temperature around 70°C or higher. Another approach is melting the fatty compound, and mixing this with a premix of cationic surfactant and water. These processes upon cooling lead to the formation of structures comprising the fatty compound and the cationic surfactants. These structures may act as carrier for conditioning compounds like silicones. Also micelles or vesicles containing fatty compound and cationic surfactants may be formed. A wide range of microstructures can be formed, as well as a wide range of viscosities, and the formation of these structures is difficult to be controlled and sensitive for changes in raw materials and process conditions. The viscosity of the products may range from 150,000 to 300,000 mPa·s, while it seems that the same process is performed to produce the products. Obviously the process do not consistently produce the same structures.

WO 2010/149437 A1 discloses hair conditioning composition based on C16-C22 anionic surfactant and C16-C22 fatty material. The conditioning composition is prepared by heating water to about 70°C, polymers, fatty alcohol, and anionic surfactant, using high speed stirring, followed by cooling to 45°C with the same speed stirring when even dispersion has been obtained. Remaining components are then added to this with moderate speed stirring. For better mixtures of different fatty amphiphiles, the different fatty materials can be co-melted together before adding into the heated water along with the anionic surfactant.

WO 2009/158439 A1 discloses a hair conditioning composition comprising cationic surfactant, a high melting point fatty compound; and an aqueous carrier; wherein the composition has a specific yield point, preferably between 33 and 80 Pa. The composition comprises a lamellar sheet structure having a spacing (which is the distance between two lamellar bilayers plus the width of one lamellar bilayer) larger than 33 nanometer. The compositions are produced at elevated temperatures using a high shear homogenizer having a rotating member.

Similarly, US 2010/0143280 discloses a method for preparing a hair conditioning composition, including a mixing step conducted by using a homogenizer having a rotating member.

WO 2005/079730 A1 discloses a method for making hair conditioners using a toothed wheel rotor/stator mixers.

US 2006/040837 A1 discloses structured liquids containing anionic surfactant, isostearic acid and cationic surfactant. The viscosity of these compositions is shear-thinning, however exact viscosities are not given.

US 6,534,457 discloses a structured liquid cleansing compositions, which may contain a cationic surfactant, in addition to an anionic surfactant. There is no indication that maximises the cationic surfactactant concentration in the formulations. The viscosity of the formulations preferably ranges from 80,000 to 300,000 cps.

### SUMMARY OF THE INVENTION

As indicated before, the process of making a structured liquid for use as hair conditioner is not very repeatable. Therefore there is a need for improved processes which can be used to consistently produce structured liquids. Moreover there is a common desire among producers to improve production methods and products, for example by decrease of energy consumption, or decrease of the concentration of active compounds in the formulation, while keeping the performance of the product at least as good as the standard product. This way resources (raw materials, energy) can be saved, additionally leading to cheaper products. One such improvement is for example increase of viscosity of the composition. By increase of viscosity, the concentration of raw materials can be decreased, while the functionality of the formulation is kept the same as with a higher raw material concentration.

Therefore it is an object of the invention to provide a method for the production of structured liquid (that can be used as hair conditioner), that leads to more efficient use of raw materials, to reduce the amount of raw materials, while keeping the same functionality of the structured liquids. It is another object of the invention to provide a process that can be used to consistently produce hair conditioner compositions of the same quality and structure. Another object of the invention is to provide a composition that does not require a large amount or high concentration of ingredients, and that nevertheless have the right consistency and viscosity to be functional as hair conditioner.

We have now determined that this objective can be met by a method wherein the structured liquid, that contains water, fatty compound and cationic surfactant and can be used as hair conditioner, is produced using a Controlled Deformation Dynamic Mixer type. By this method structured liquids for use as hair conditioner can be produced that do not require high concentrations of actives, and have a good consistency and viscosity to be functional as hair conditioner.

Accordingly in a first aspect the invention provides a method for production of a structured liquid composition comprising water, a fatty compound, and a cationic surfactant, comprising the step:
a) melting the fatty compound and mixing it with a mixture of cationic surfactant and water, or melting fatty compound and mixing it with the cationic surfactant, and mixing this mixture with water;
   characterised in that in a next step
b) the mixture from step a) is introduced into a distributive and dispersive mixing apparatus of the Controlled Deformation Dynamic Mixer type,
   wherein the mixer is suitable for inducing extensional flow in a liquid composition, and wherein the mixer comprises closely spaced relatively moveable confronting surfaces at least one having a series of cavities therein in which the cavities on each surface are arranged such that, in use, the cross-sectional area for flow of the liquid successively increases and decreases by a factor of at least 5 through the apparatus. In a second aspect the present invention provides a structured liquid obtainable by the method according to the invention. The second aspect of the invention provides a structured liquid containing fatty compound at maximally 3% by weight, and cationic surfactant at maximally 2% by weight, and water, and wherein the structured liquid has a dynamic viscosity of at least 190,000 mPa·s, preferably at least 220,000 mPa.s, measured at a shear rate lower than 0.5 s⁻¹ and a temperature of 20°C.

In a third aspect the present invention provides use of a structured liquid prepared according to the method of the first aspect of the invention or according to the composition of the second aspect of the invention as hair conditioner.

### DESCRIPTION OF FIGURES

Figure 1: Schematic representation of a Cavity Transfer Mixer (CTM); 1: stator, 2: annulus; 3: rotor; with cross-sectional views below.
Figure 2: Schematic representation of a Controlled Deformation Dynamic Mixer (CDDM) ; 1: stator, 2: annulus; 3: rotor; with cross-sectional views below.
Figure 3: Schematic representation of a preferred embodiment of the CDDM apparatus, cross-sectional view (direction of bulk flow preferably from left to right).
Figure 4: Schematic representation of a preferred embodiment of the CDDM apparatus, cross-sectional view (direction of bulk flow preferably from left to right).

### DETAILED DESCRIPTION OF THE INVENTION

Unless defined otherwise, all technical and scientific terms used herein have the same meaning as commonly understood by one of ordinary skill in the art.

All percentages, unless otherwise stated, refer to the percentage by weight. The abbrevation 'wt%' or'% (w/w)' refers to percentage by weight.

In case a range is given, the given range includes the mentioned endpoints.

Ambient temperature is considered to be a temperature between 15°C and 25°C, preferably between 17°C and 25°C, preferably between 20°C and 23°C

### Cavity Transfer Mixers (CTMs)

Similar as in WO 96/20270, CTMs are defined as mixers comprising confronting surfaces, at least one of the surfaces, preferably both surfaces, having a series of cavities formed therein in which the surfaces move relatively to each other and in which a liquid material is passed between the surfaces and flows along a pathway successively through the cavities in each surface. The cavities are arranged on the relevant surfaces such that shear is applied to the liquid as it flows between the surfaces. The cavities are arranged on the respective surfaces such that there is a relatively small change in the effective cross sectional flow area as the material passes through the mixer. In such mixers, primarily distributive mixing is obtained. Generally the cross-sectional area for flow varies by a factor of less than 3 through the apparatus. Shear is applied by the relative movement of the surfaces in a generally perpendicular direction to the flow of material there between.

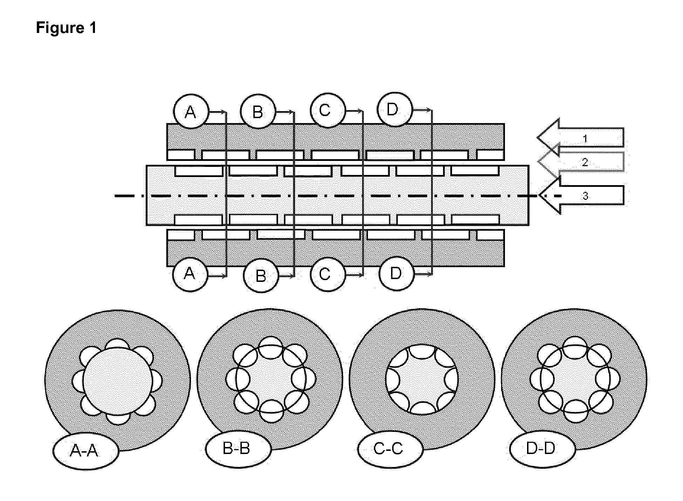

Here we exemplify CTMs by reference to Figure 1 which displays an axial section and four transverse radial sections through a CTM configured as a 'concentric cylinder' device and comprising an inner rotor journalled within an outer stator. Briefly, the axial section shows the relative axial positions of rotor and stator cavities which are time invariant, whereas the transverse sections (A-A, B-B, C-C, D-D) demonstrate the axial variation in the available cross-sectional area for material flow axially:
▪ A-A through the stator cavities in positions in which those stator cavities are confronted by 'rotor rings', ie the circumferentially extending rings which separate successive rings of rotor cavities;
▪ B-B between the stator cavities and the rotor cavities in positions in which the former are confronted by the latter;
▪ C-C through the rotor cavities in positions in which those rotor cavities are confronted by 'stator rings', ie the circumferentially extending rings which separate successive rings of stator cavities;
▪ D-D between the rotor cavities and the stator cavities in positions in which the former are confronted by the latter.

The key feature to note is that there is little variation in the cross-sectional area for flow as the material passes axially down the device.

### Controlled Deformation Dynamic Mixers (CDDMs)

Similar as in WO 96/20270, CDDMs are distinguished from CTMs by their description as mixers: comprising confronting surfaces, at least one of the surfaces, preferably both surfaces, having a series of cavities formed therein in which the surfaces move relatively to each other and in which a liquid material is passed between the surfaces and flows along a pathway successively through the cavities in each surface and is subjected to extensional deformation and/or shear deformation and preferably both extensional and shear deformation.The cavities are arranged on the relevant surfaces such that shear is applied by the relative movement of the surfaces in a generally perpendicular direction to the flow of material there between. In addition to shear, significant extensional flow and efficient distributive and dispersive mixing may be secured by providing an apparatus having confronting surfaces and cavities therein in which the cavities are arranged such that the cross sectional area for flow of the liquid successively increases and decreases by a factor of at least 5 through the apparatus.

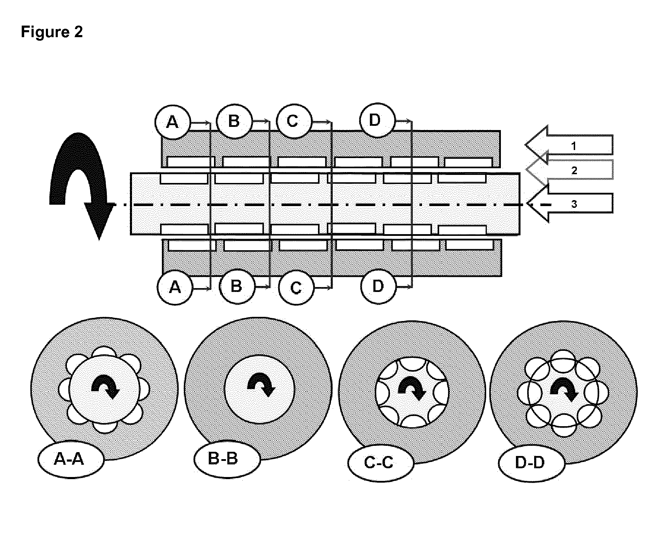

Here we exemplify CDDMs by reference to Figure 2 which displays an axial section and four transverse radial sections through a CDDM configured as a 'concentric cylinder' device comprising an inner rotor journalled within an outer stator. Briefly, the axial section shows the relative axial positions of rotor and stator cavities which are time invariant, whereas the transverse sections (A-A, B-B, C-C, D-D) demonstrate the axial variation in the available cross-sectional area for material flow axially :
▪ A-A through the stator cavities in positions in which those stator cavities are confronted by 'rotor rings', ie the circumferentially extending rings which separate successive rings of rotor cavities;
▪ B-B between the stator cavities and the rotor cavities through the annulus formed in those positions in which the 'rotor rings' are confronted by the 'stator rings';
▪ C-C through the rotor cavities in positions in which those rotor cavities are confronted by 'stator rings', ie the circumferentially extending rings which separate successive rings of stator cavities;
▪ D-D between the rotor cavities and the stator cavities in positions in which the former are confronted by the latter.

Clearly there is a significant variation in the cross-sectional area for flow as the material passes axially through the annulus formed between the 'rotor rings' and the 'stator rings' (BB), and between confronting rotor cavities and stator cavities (D-D).

By comparison of Figure 1 and Figure 2, it will be understood that CDDMs are distinguished from CTMs by the relative position of the rotor and stator and consequent incorporation of an extensional component of flow. Hence CDDMs combine the distributive mixing performance of CTMs with the dispersive mixing performance of multiple expansion-contraction static mixers.

Although the CDDM has a moving part, it may also be run in static mode, meaning that one or more fluids are pumped through the apparatus without rotation of the rotor. This is called the static mode of the apparatus.

### Method for production of structured liquid

In a first aspect the invention provides a method for production of a structured liquid composition comprising water, a fatty compound, and a cationic surfactant, comprising the step:
a) melting the fatty compound and mixing it with a mixture of cationic surfactant and water, or melting fatty compound and mixing it with the cationic surfactant, and mixing this mixture with water;
   characterised in that in a next step
b) the mixture from step a) is introduced into a distributive and dispersive mixing apparatus of the Controlled Deformation Dynamic Mixer type,
   wherein the mixer is suitable for inducing extensional flow in a liquid composition, and wherein the mixer comprises closely spaced relatively moveable confronting surfaces at least one having a series of cavities therein in which the cavities on each surface are arranged such that, in use, the cross-sectional area for flow of the liquid successively increases and decreases by a factor of at least 5 through the apparatus.

For the purposes of understanding the operation of the CDDM in general, the disclosure of WO 96/20270 is incorporated herein by reference. Regions of distributive mixing (where the flow path is wide) comprises CTM-like cavities moving across each other in a direction perpendicular to the bulk flow of liquid. Between these regions of distributive mixing are regions in which the flow path is narrower and the flow is more extensional. It is possible for a mixer used in the method according to the invention to be provided with one on more regions in which the juxtaposition is such that the arrangement is CTM-like and one or more regions in which the arrangement is CDDM-like.

In step a) a premix is made of the ingredients of the composition. In one possible way to make the premix, the fatty compound is melted and mixed with a mixture of cationic surfactant and water. In this case, the fatty compound is melted at a temperature higher than the melting point of the fatty compound, typically at a temperature of at least 70°C, preferably at least 75°C. The maximum melting temperature in this step preferably is 110°C. The cationic surfactant is dispersed in at least part of the water of the total formulation, and this mixture preferably also has an elevated temperature, which is similar to the temperature of the molten fatty compound. Hence preferably the temperature of the aqueous phase is at least 70°C, preferably at least 75°C, and preferably lower than 100°C. Preferably, the temperature of the mixing in step a) is at least 70°C. The two phases (molten fatty compound and mixture of cationic surfactant and water) are preferably mixed in a vessel under high shear, for example by using a Silverson high shear mixer (ex Silverson Machines Ltd., Chesham, Buckinghamshire, UK), operated at a rotational speed of preferably 3,000-5,000 rpm, which preferably generates a shear rate of 40,000-50,000 s⁻¹.

The mixture may also be cooled by further addition of water, which is at a low temperature, for example 20-50°C, preferably at 20-40°C. The water may contain the other ingredients of the compositions like silicones, preservative, perfume, and any other ingredient. The water and further ingredients are then gently mixed into the composition.

Preferably, other ingredients like silicones, preservative, perfume, and any other ingredient are added to the composition after the temperature of the mixture has decreased to a temperature ranging from 40 to 50°C, preferably at about 45°C. The ingredients are then gently mixed into the composition.

Subsequently this mixture is brought into the CDDM mixer, to perform mixing step b). Summarising, this process can be described by the following steps:
1. preparing a mixture of cationic surfactant and water;
2. mixing molten fatty compound into this mixture, preferably using high shear mixer;
3. optionally adding further ingredients;
4. mixing this mixture in CDDM apparatus.

These steps 1, 2, and 3 together form step a) of the method of the invention, and this step 4 forms step b) of the method of the invention.

Optionally, after step b) further water is added to the mixture obtained from step b).

Alternatively, the premix in step a) is made by melting fatty compound and mixing it with the cationic surfactant, and mixing this mixture with water. Preferably the fatty compound and cationic surfactant are melted at a temperature higher than the melting point of the fatty compound, typically at a temperature of at least 70°C, preferably at least 75°C. The maximum melting temperature in this step preferably is 110°C. The fatty compound and cationic surfactant may be mixed using a high pressure homogeniser, e.g. a Sonolator from Sonic Corporation (Stratford, CT, USA), operated at a pressure of preferably 10 to 30 bar. Preferably water at a similar temperature of at least 70°C, preferably at least 75°C, and preferably lower than 100°C is added to the mixture of cationic surfactant and fatty compound, while mixing in the homogeniser, to produce a homogeneous mixture of fatty compound, cationic surfactant, and water. The water added to the high pressure homogeniser preferably is about half of the water required in the formulation, preferably is 50% by weight of the total water required.

Subsequently this mixture obtained from the high pressure homogeniser may be further mixed using a high shear mixer. Prior to mixing in the high shear mixer, the remaining part of the water, preferably 50% by weight of the total water required, is added to the mixture from the homogeniser. The temperature of the water added in this step preferably ranges from 20 to 50°C, more preferred from 20 to 40°C. The high shear mixing step then produces a homogeneous emulsion. This high shear mixing step may be performed for example by using a Silverson high shear mixer (ex Silverson Machines Ltd., Chesham, Buckinghamshire, UK), operated at a rotational speed of preferably 3,000-5,000 rpm, which preferably generates a shear rate of 40,000-50,000 s⁻¹. Preferably, the other ingredients like silicones, preservative, perfume, and any other ingredient are added to the composition together with the remaining water. Subsequently this mixture is brought into the CDDM mixer, to perform mixing step b).

Summarising, this process can be described by the following steps:
1. preparing a mixture of cationic surfactant and fatty compound;
2. mixing part of the water with this mixture, preferably using a high pressure homogeniser (like Sonolator);
3. adding remaining water and optionally further ingredients, and mixing this in a high shear mixer (like Silverson);
4. mixing this mixture in CDDM apparatus.

These steps 1, 2, and 3 together form step a) of the method of the invention, and this step 4 forms step b) of the method of the invention.

Optionally, after step b) further water is added to the mixture obtained from step b).

### Cationic Surfactants

The compositions of the inventions comprise a cationic surfactant. Suitable conditioning surfactants include those selected from cationic surfactants, used singly or in admixture. Preferably, the cationic surfactants have the formula N⁺R¹R²R³R⁴ wherein R¹, R², R³ and R⁴ are independently (C₁ to C₃₀) alkyl or benzyl. Hence, preferably the cationic surfactant comprises a quaternary ammonium moiety. Preferably, one, two or three of R¹, R², R³ and R⁴ are independently (C₄ to C₃₀) alkyl and the other R¹, R², R³ and R⁴ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or-COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as Genamin CTAC, ex Hoechst Celanese. Another particularly useful cationic surfactant for use in conditioners according to the invention is behenyltrimethylammonium chloride, available commercially, for example as Genamin KDMP, ex Clariant.

Another example of a class of suitable cationic surfactants for use in the invention, either alone or together with one or more other cationic surfactants, is a combination of (i) and (ii) below:
(i) an amidoamine corresponding to the general formula (I):

   R¹-CONH(CH₂)ₘN(R²)R³

   in which R¹ is a hydrocarbyl chain having 10 or more carbon atoms,
   R² and R³ are independently selected from hydrocarbyl chains of from 1 to 10 carbon atoms, and m is an integer from 1 to 10; and
(ii) an acid.

As used herein, the term hydrocarbyl chain means an alkyl or alkenyl chain.

Preferred amidoamine compounds are those corresponding to formula (I) in which R¹ is a hydrocarbyl residue having from 11 to 24 carbon atoms,

R² and R³ are each independently hydrocarbyl residues, preferably alkyl groups, having from 1 to 4 carbon atoms, and m is an integer from 1 to 4.

Preferably, R² and R³ are methyl or ethyl groups. Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyldimethylamine, arachidamidopropyldiethylamine, arachid-amidoethyldiethylamine, arachidamidoethyldimethylamine, and mixtures thereof. Particularly preferred amidoamines useful herein are stearamidopropyldimethylamine, stearamidoethyldiethylamine, and mixtures thereof.

Commercially available amidoamines useful herein include:
stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

A protonating acid may be present. Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudoquaternary ammonium cationic surfactant.

Suitably, the acid is included in a sufficient amount to protonate more than 95 mole% (293 K) of the amidoamine present.

Also combinations of cationic surfactants may be used.

In conditioners of the invention, the level of cationic surfactant will generally range from 0.01 % to 10%, more preferably 0.05 % to 7.5%, most preferably 0.1 % to 5% by weight of the composition. Preferably the concentration of the one or more cationic surfactants is maximally 2% by weight, preferably 1.5% by weight, preferably 1% by weight.

### Fatty compound

Compositions according to the present invention comprise a fatty compound, that usually is a dispersed, non-volatile, water-insoluble, non-silicone, oily conditioning agent. By 'insoluble' is meant that the material is not soluble in water (distilled or equivalent) at a concentration of 0.1 % (w/w), at 25°C.

Suitable oily or fatty materials are selected from hydrocarbon oils, fatty esters and mixtures thereof. Straight chain hydrocarbon oils will preferably contain from 12 to 30 carbon atoms. Also suitable are polymeric hydrocarbons of alkenyl monomers, such as C₂-C₆ alkenyl monomers. Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and tri-esters of glycerol and long chain carboxylic acids such as C₁-C₂₂ carboxylic acids. Preferred materials include cocoa butter, palm stearin, sunflower oil, soyabean oil and coconut oil.

Most preferred the fatty compound comprises a fatty alcohol, a fatty acid, a fatty alcohol derivative, or a fatty acid derivative, or mixtures thereof. These fatty compounds preferably have a melting point of 25°C or higher, preferably 40°C or higher, more preferably 45°C or higher, still more preferably 50°C or higher, in view of stability of the emulsion especially the gel matrix. Preferably, such melting point is up to about 90°C, more preferably up to about 80°C, still more preferably up to about 70°C, even more preferably up to about 65°C.

Preferably the fatty compound comprises a fatty alcohol. Fatty alcohols are typically compounds containing straight chain alkyl groups. The combined use of fatty alcohols and cationic surfactants in conditioning compositions is believed to be especially advantageous, because this leads to the formation of a lamellar phase, in which the cationic surfactant is dispersed. The fatty alcohols useful herein are those having from 14 to 30 carbon atoms. Preferably, the fatty compound comprises a C16-C22 fatty alcohol. These fatty alcohols are saturated and can be straight or branched chain alcohols.

Preferred fatty alcohols include, for example, cetyl alcohol (hexadecan-1-ol, having a melting point of about 56°C), stearyl alcohol (1-octadecanol, having a melting point of about 58-59°C), behenyl alcohol (having a melting point of about 71 °C), and mixtures thereof. In the present invention, more preferred fatty alcohols are cetyl alcohol, stearyl alcohol and mixtures thereof. The use of these materials is also advantageous in that they contribute to the overall conditioning properties of compositions of the invention. The level of fatty compound, preferably fatty alcohol, preferably C16-C22 fatty alcohol, in structured liquids prepared according to the invention will generally range from 0.01 to 10%, preferably from 0.1 % to 8%, more preferably from 0.2 % to 7 %, most preferably from 0.3 % to 6 % by weight of the composition. Preferably the concentration of fatty compound, preferably fatty alcohol, preferably C16-C22 fatty alcohol, is maximally 3% by weight, preferably 2.5% by weight, preferably 1.5% by weight. Preferably, the weight ratio of cationic surfactant to fatty compound ranges from 1:1 to 1:10, preferably from 1:1.5 to 1:8, preferably from 1:2 to 1:5. If the weight ratio of cationic surfactant to fatty alcohol is too high, this can lead to eye irritancy from the composition. If it is too low, it can make the hair feel squeaky for some consumers. The structured liquids made according to the method of the invention may contain other common ingredients.

### Silicones

The compositions of the invention can contain emulsified droplets of a silicone conditioning agent, for enhancing conditioning performance. Preferably, a silicone conditioning agent is added to the mixture from step a) prior to introducing the mixture into step b), at a concentration ranging from 0.01% to 10% by weight of the total composition.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The kinematic viscosity of the emulsified silicone itself (not the emulsion or the final hair conditioning composition) is typically at least 0.01 m².s⁻¹ at 25 °C. The kinematic viscosity of the silicone itself is preferably at least 0.06 m².s⁻¹, most preferably at least 0.5 m².s⁻¹, ideally at least 1 m².s⁻¹. Preferably the kinematic viscosity does not exceed 1,000 m².s⁻¹ for ease of formulation.

Emulsified silicones for use in the compositions of the invention will typically have an size in the composition of less than 30, preferably less than 20, more preferably less than 15 micrometer. Preferably the average silicone droplet is greater than 0.5 micrometer, more preferably greater than 1 micrometer, ideally from 2 to 8 micrometer.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

The total amount of silicone is preferably from 0.01 wt% to 10 %wt of the total composition more preferably from 0.1 wt% to 5 wt%, most preferably 0.5 wt% to 3 wt% is a suitable level.

Preferably, the composition comprises vegetable oils. Preferred vegetable oils include jojoba, soybean, sunflower seed oil, rice bran, avocado, almond, olive, sesame, castor, coconut, mink oils

Preferably, the composition comprises anionic polymer. Examples of anionic polymer include alkyl vinyl ether-maleic acid copolymers (Trade name: Gantrez S95), Xanthan Gum (Trade Name: Keltrol RD) and Dehydroxanthan gum (Trade Name: Amaze XT).

Preferably, the composition has a pH of from 2.5 to 8.

Preferably the compositions made according to the method of the invention comprise at least 90% by weight water, preferably at least 92% by weight, preferably at least 95% by weight.

### Mixing in Controlled Deformation Dynamic Mixer

The mixture from step a) is preferably introduced into the Controlled Deformation Dynamic Mixer type at a temperature ranging from 10 to 40°C. Preferably, the mixture from step a) is introduced into the mixing apparatus at a temperature ranging from 20 to 35°C. Even more preferred, the temperature at which the mixture from step a) is introduced into the mixing apparatus ranges from 20 to 30°C. The shear rate in the mixing apparatus is in the order of magnitude of at least 200,000 s⁻¹.

Alternatively, the mixture comprising water, cationic surfactant and fatty compound introduced into the mixing apparatus may have a temperature ranging from 40 to 80°C, preferably 50 to 70°C.

An advantage of the CDDM mixing device used in the method of the invention is that elongational and/or rotational shear flows can be controlled well, by modification of the rotational speed of one surface relative to the other. Moreover, also the distance between the two surfaces can be designed such that the flow field can be modified and adapted to the needs of the product to be produced by the CDDM.

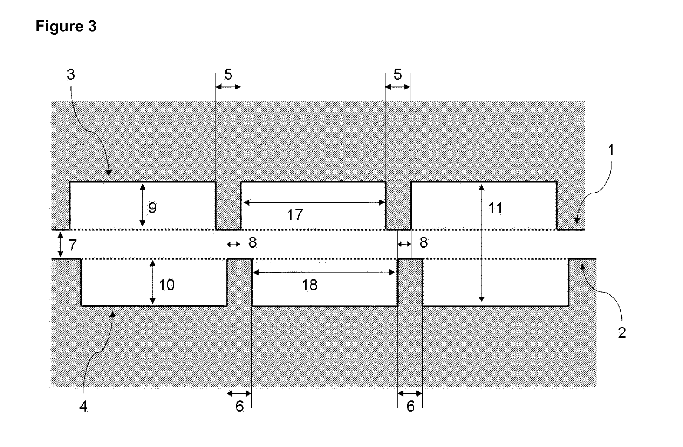

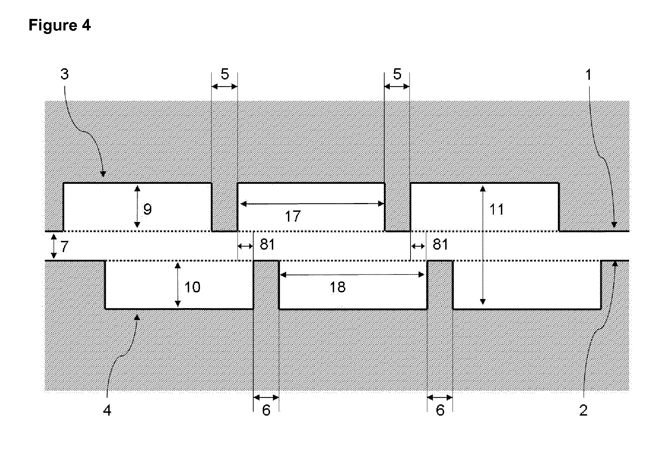

In a preferred embodiment the CDDM apparatus can be described by the following. With reference to Figure 3 and Figure 4, preferably the Controlled Deformation Dynamic Mixer comprises two confronting surfaces (1, 2), spaced by a distance (7), wherein the first surface (1) contains at least three cavities (3), wherein at least one of the cavities has a depth (9) relative to the surface (1),
wherein the second surface (2) contains at least three cavities (4) wherein at least one of the cavities has a depth (10) relative to the surface (2),
wherein the cross-sectional area for flow of the liquid available during passage through the apparatus successively increases and decreases at least 3 times, and wherein the surface (1) has a length (5) between two cavities, and wherein the surface (2) has a length (6) between two cavities, and
wherein the surfaces (1, 2) are positioned such that the corresponding lengths (5, 6) overlap to create a slit having an offset distance (8) or do not overlap creating a offset distance (81),
wherein the cavities are arranged such that the cross-sectional area for flow of the liquid available during passage through the apparatus successively increases in the cavities and decreases in the slits by a factor of at least 5 and
wherein the distance (7) between the two surfaces (1,2) is between 2 micrometer and 300 micrometer, and wherein
either the ratio between the offset distance (8) and the distance (7) between the two surfaces (1, 2) ranges from 0 to 250,
or wherein the ratio between the offset distance (81) and the distance (7) between the two surfaces (1, 2) ranges from 0 to 30.

With reference to Figure 3 and Figure 4: there are several possible configurations for the mixing apparatus. In one preferred combination the confronting surfaces 1, 2 are cylindrical. In such a configuration the apparatus will generally comprise a cylindrical drum and co-axial sleeve. The confronting surfaces 1, 2 will be defined by the outer surface of the drum and the inner surface of the sleeve. However, there are alternative configurations in which the confronting surfaces are circular or disk-shaped. Between these two extremes of configuration are those in which the confronting surfaces are conical or frusto-conical. Non-cylindrical embodiments allow for further variation in the shear in different parts of the flow through the mixer.

The regions where the confronting surfaces 1, 2 are most closely spaced are those where the shear rate within the mixer tends to be the highest. The slit 7 between the surfaces between the confronting surfaces 1, 2 forms this region, combined with offset distance 8 or offset distance 81. High shear contributes to power consumption and heating. This is especially true where the confronting surfaces of the mixer are spaced by a gap of less than around 50 micrometer. Advantageously, confining the regions of high shear to relatively short regions means that the power consumption and the heating effect can be reduced, especially where in the CTM-like regions the confronting surfaces are spaced apart relatively widely.

Hence the apparatus can be designed such that good mixing is obtained, while keeping the pressure drop over the apparatus as small as possible. The design can be modified by adjusting the dimensions of the various parts of the apparatus, as explained in the following.

The distance 7 between the corresponding surfaces preferably is from 2 micrometers to 300 micrometers, which corresponds to the height of the slit. Preferably the distance 7 is between 3 micrometer and 200 micrometer, preferably between 5 micrometer and 150 micrometer, preferably between 5 micrometer and 100 micrometer, preferably between 5 micrometer and 80 micrometer, preferably between 5 and 60 micrometer, preferably between 5 micrometer and 40 micrometer. More preferably the distance 7 is between 8 micrometer and 40 micrometer, more preferably between 8 micrometer and 30 micrometer, more preferably between 10 micrometer and 30 micrometer, more preferably between 10 micrometer and 25 micrometer, more preferably between 15 micrometer and 25 micrometer.

The actual height of the slit 7 depends on the dimensions of the apparatus and the required flow rate, and the skilled person will know how to design the apparatus such that the shear rates within the apparatus remain relatively constant irrespective of the size of the apparatus.

The surfaces 1 and 2 that each contain at least three cavities 3, 4 create a volume between the surfaces for flow of the two fluids which are mixed. The cavities in the surface effectively increase the surface area available for flow. Due to the presence of the cavities, the small area for flow between the surfaces 1 and 2 can be considered to be a slit having a height 7. The spacing 5 between two cavities in surface 1 and spacing 6 between two cavities in surface 2 and the relative position of these corresponding parts (the offset) determine the maximum length or offset distance 8 of the slit (in the direction of bulk liquid flow). The maximum length of the slit is equal to the smallest of the spacings 5, 6.

Preferably, the two surfaces 1, 2 with cavities 3, 4, that together form the volume for the mixing of the three phases (aqueous phase, liquid oil, and structuring fat), are positioned such that the corresponding spacings 5, 6 of the surfaces (that create the length of the slit) create an offset distance 8 of the slit (in the direction of the bulk flow) which is maximally 250 times as large as the distance 7 between the surfaces. The two surfaces 1, 2 can be positioned such that offset distance 8 can be adjusted. Preferably the ratio between the offset distance 8 and the distance 7 between the two surfaces 1, 2 ranges from 0 to 100, preferably 0 to 10, preferably 0 to 5. Most preferably the ratio between the offset distance 8 and the distance 7 ranges from 0 to 1. As an example, when the ratio between offset distance 8 and distance 7 is 5, and the distance 7 between the two surfaces 1, 2 is 15 micrometer, then the offset distance 8 of the slit is 75 micrometer.

Preferably and alternatively the surfaces 1, 2 are positioned such that no overlap is created, however in that case an offset distance 81 is created. In that case there is no overlap between the corresponding parts of the surfaces 1, 2, and the slit is created with what could be called a 'negative overlap'. The two surfaces 1, 2 can be positioned such that offset distance 81 can be adjusted. The ratio between the offset distance 81 and the distance 7 between the two surfaces 1, 2 preferably ranges from 0 to 30. This 'negative overlap' accommodates the possibility of near zero distance 7 between the two corresponding surfaces 1 and 2. Preferably the offset distance 81 is such, that the ratio between the offset distance 81 and the distance 7 between the two surfaces 1, 2 ranges from 0 to 15, more preferred from 0 to 10, preferably from 0 to 5, preferably from 0 to 2 and more preferably from 0 to 1. Alternatively and preferably the offset distance 81 is maximally 600 micrometer, more preferably maximally 300 micrometer. As an example, when the ratio between length 81 and distance 7 is 2, and the distance 7 between the two surfaces 1, 2 is 15 micrometer, then length 81 (or what could be called negative overlap) is 30 micrometer.

A further benefit of this variation in the normal separation of the confronting surfaces in the direction of bulk flow, is that by having relatively small regions of high shear, especially with a low residence time is that the pressure drop along the mixer can be reduced without a compromise in mixing performance.

The little overlap (meaning that offset distance 8 approaches zero, or that the mixing apparatus has a 'negative overlap' or offset distance 81) between the corresponding parts of the surfaces 1, 2 leads to a relatively small pressure that is required in order to create a fine dispersion, as compared to apparatuses which have a longer overlap and consequently also need a higher pressure. Usually a longer distance of a slit (or longer capillary) leads to smaller droplets of the dispersed phase. Now we found that with a short capillary or even without capillary the droplets of the dispersed phase remains small, while the pressure required is relative low, as compared to a longer overlap. For example high pressure homogenisers may operate at pressures up to 1,600 bar or even higher. Hence preferably in the method of the invention, the mixing apparatus is operated at a pressure less than 200 bar, preferably less than 80 bar, preferably less than 60 bar, preferably less than 40 bar, most preferred less than 30 bar. With these relatively low pressures a good mixing process is obtained.

An additional advantage of the relatively low pressure is that the energy consumption for applying the pressure is much lower than in devices like high pressure homogeniser which may use pressures of up to 1,000 bar. Moreover less stringent material specifications for design of an apparatus to withstand high pressures is required, such that raw materials can be saved.

With reference to Figure 3 and Figure 4, the fluids preferably flow from left to right through the apparatus. The slits create an acceleration of the flow, while at the exit of the slit the fluids decelerate due to the increase of the surface area for flow and the expansion which occurs. The acceleration and deceleration leads to the break up of the large droplets of the dispersed phase, to create finely dispersed droplets in a continuous phase. Droplets that are already small, remain relatively untouched. The flow in the cavities is such that the droplets of the dispersed phase eventually become evenly distributed in the continuous phase.

The cross-sectional area for flow of the liquid available during passage through the apparatus successively increases and decreases at least 5 times, and these passages lead to effective mixing of the two fluids. This means that the cross-sectional area for flow of liquid in the cavities is at least 5 times larger than the cross-sectional area for flow of liquid in the slits. This relates to the ratio between distance 11 and distance 7. Preferably the cross-sectional area for flow is designed such that the cross-sectional area for flow of the liquid available during passage through the apparatus successively increases and decreases by a factor of at least 7, preferably at least 10, preferably at least 25, preferably at least 50, up to preferred values of 100 to 400. The cross-sectional surface area for flow of the fluids is determined by the depth 9 of the cavities 3 in the first surface 1 and by the depth 10 of the cavities 4 in the second surface 2. The total cross-sectional area is determined by the distance 11 between the bottoms of two corresponding cavities in the opposite surfaces.

The surfaces 1, 2 each contain at least three cavities 3, 4. In that case the flow expands at least 3 times during passage, and the flow passes through at least 3 slits during the passage. Preferably the cross-sectional area for flow of the liquid available during passage through the apparatus successively increases and decreases between 4 and 8 times. This means that the flow during passage experiences the presence of between 4 and 8 slits and cavities.

The shape of the cavities 3 may take any suitable form, for example the cross-section may not be rectangular, but may take the shape of for example a trapezoid, or a parallelogram, or a rectangle where the corners are rounded. Seen from above, the cavities may be rectangular, square, or circular, or any other suitable shape. Any arrangement of the cavities and the number of cavities and size of the cavities may be within the scope of the present invention.

The mixing apparatus preferably is operated dynamically, meaning that one of the surfaces rotates relative to the other. In that case preferably one of the surfaces is able to rotate relative to the other surface at a frequency between 1,000 and 25,000 rotations per minute, preferably between 3,000 and 12,000 rotations per minute.

As indicated before , in another preferred embodiment, the surfaces are static with respect to each other. That means that during the mixing operations the liquid is pressed through the mixing apparatus, and the surfaces do not rotate.

In general rotation may lead to improved mixing process. Static operation has the advantage that less energy is required for mixing. Operation of the device without rotation leads to very efficient and effective mixing of fluids. The static operation enjoys the major advantage of potentially easier deployment and less mechanical complexity and possibly wear of equipment. The dynamic operation has the advantage that the required pressure to pump one or more fluids through the device, is lower than at the static operation.

Additional features of the known CTM and CDDM may be incorporated in the mixer described herein. For example, one or both of the confronting surfaces may be provided with means to heat or cool it. Where cavities are provided in the confronting surfaces these may have a different geometry in different parts of the mixer to as to further vary the shear conditions.

In a preferred example, the dimensions of such a CDDM apparatus used in the invention are such that the distance between the two surfaces 7 is between 10 and 20 micrometer; and/or wherein the length of the slit 8 is maximally 2 millimeter, for example 80 micrometer, or 20 micrometer, or even 0 micrometer. The length of the slit 8 plus the length of the cavity 17, 18 combined is maximally 10 millimeter; and/or wherein the depth of the cavities 9, 10 is maximally 2 millimeter. In that case preferably the internal diameter of the outer surface is between 20 and 30 millimeter, preferably about 25 millimeter. The total length of the apparatus in that case is between 7 and 13 centimeter, preferably about 10 centimeter. The length means that this is the zone where the fluids are mixed. The rotational speed of such a preferred apparatus is preferably 0 (static), or more preferred alternatively between 5,000 and 25,000 rotations per minute.

The shape of the area for liquid flow may take different forms, and naturally depends on the shape of the confronting surfaces. If the surfaces are flat, then the cross-sectional area for flow may be rectangular. The two confronting surfaces may also be in a circular shape, for example a cylindrical rotor which is positioned in the centre of a cylindrical pipe, wherein the outside of the cylindrical rotor forms a surface, and the inner surface of the cylindrical pipe forms the other surface. The circular annulus between the two confronting surface is available for liquid flow.

### Structured liquids for use as hair conditioner

In a second aspect the present invention provides a structured liquid obtainable by the method according to the invention. The second aspect of the invention provides a structured liquid containing fatty compound at maximally 3% by weight, and cationic surfactant at maximally 2% by weight, and water, and wherein the structured liquid has a dynamic viscosity of at least 190,000 mPa·s, preferably at least 220,000 mPa·s, measured at a shear rate lower than 0.5 s⁻¹ and a temperature of 20°C.

The advantage of the method is that structured liquids are produced that are relatively low in ingredients, while still being relatively high in viscosity. This means that can be saved on the amount of raw materials and resources required to make good and functional compositions. The compositions may be used for rinse-off treatments, meaning that the composition is rinsed off the scalp, usually within a few minutes after applying it to the scalp. The compositions may also be left on the head, i.e. not rinsed off after application, known as leave-on treatment. The leave-on treatments usually have a higher viscosity than the rinse-off treatments.

Preferably, the fatty compound comprises a fatty alcohol, preferably a C16-C22 fatty alcohol. Preferred aspects of the fatty compounds as disclosed herein in the context of the first aspect of the invention, will apply to the second aspect of the invention as well. Preferred ingredients of the compositions made according to the method of the invention, are preferred ingredients of the compositions according to the second aspect of the invention as well.

Preferably the compositions of the invention comprise at least 90% by weight water, preferably at least 92% by weight, preferably at least 95% by weight. Preferably the concentration of fatty compound is maximally 3% by weight, preferably 2.5% by weight, preferably 1.5% by weight. Preferably the concentration of the one or more cationic surfactants is maximally 2% by weight, preferably 1.5% by weight, preferably 1% by weight. If present, the concentration of silicone conditioning agent preferably ranges from 1 to 4% by weight.

Preferably the dynamic viscosity of the composition according to the second aspect of the invention is at least 190,000 mPa·s (190 Pa·s), preferably at least 200,000 mPa·s, preferably at least 220,000 mPa·s. Even more preferred the dynamic viscosity of the composition is at least 250,000 mPa·s or even 300,000 mPa·s. Preferably the dynamic viscosity is maximally 500,000 mPa·s, preferably maximally 400,000 mPa·s. These dynamic viscosities are determined at a shear rate lower than 0.5 s⁻¹ and a temperature of 20°C.

Alternatively the composition according to the invention can be characterised as a structured liquid containing a fatty compound, cationic surfactant and water, wherein the ratio of the dynamic viscosity of the liquid to the mass fraction of the fatty compound is at least 1.5x10⁷, preferably at least 3x10⁷, preferably at least 4x10⁷, wherein the viscosity is expressed in units of mPa.s and is measured at a shear rate of less than 0.5 s⁻¹ and at a temperature of 20°C and the mass fraction is expressed in the unit of mass of the fatty compound per unit mass of the total composition.

The dynamic viscosities can be determined using a Brookfield RV viscometer (ex Brookfield Engineering Laboratories, Inc., Middleboro, MA, USA), fitted with a T-Bar B spindle and operated at a rotational speed of 0.5 rpm, and at a temperature of 20°C. The shear rate in that case is lower than 1 s⁻¹, or even lower than 0.5 s⁻¹.

In a third aspect the present invention provides use of a structured liquid prepared according to the method of the first aspect of the invention or according to the composition of the second aspect of the invention as hair conditioner.

### EXAMPLES

The following non-limiting examples illustrate the present invention.

### CDDM Apparatus

Experiments were carried out in a CDDM apparatus as schematically depicted in Figure 2 and Figure 3, wherein the apparatus comprises a stainless steel cylindrical drum and co-axial sleeve (the confronting surfaces 1, 2 are cylindrical). The confronting surfaces 1, 2 are defined by the outer surface of the drum and the inner surface of the sleeve, respectively. The CDDM can be described by the following parameters:
- slit height 7 is 35-40 micrometer;
- offset distance 8 is 20 micrometer;
- total length of the apparatus is 10 centimeter (length means the zone where the fluids are mixed);
- across the length of the CDDM in axial direction (in flow direction) the flow experiences six slits with height 7, the flow is contracted 6 times;
- depth 9, 10 of cavities 3, 4 is maximally 2 millimeter;
- internal diameter of the stator is 25 millimeter;
- rotational speed of the apparatus is up to 25,000 rotations per minute, and it was operated in these experiments at 2,000 to 18,000 rotations per minute;

**Table 1 Raw Material Specifications.**

| *Ingredient name* | *Chemical name* | *Supplier* | *Functionality* |
|---|---|---|---|
| Genamin BTLF (70% active in di propylene glycol) | Alkyltrimethyl ammonium chloride or Behenyltrimonium chloride (melting point about 65-70°C) | Clariant | Cationic surfactant |
| Lexamine S-13 (100%) | stearamidopropyldimethylamine | Inolex (Philadelphia, PA, USA) | Cationic surfactant |
| Lanette S3 (100%) | Cetyl stearyl alcohol (mixture of cetyl alcohol and stearyl alcohol) | Cognis | Fatty alcohol |
| Purac HS88 (88%) | Lactic acid | Purac (Gorinchem, Netherlands) | pH modifier |
| Potassium chloride (100%) | | | Viscosity modifier |
| Nipagin (100%) | Methylparaben (methyl 4-hydroxy benzoate) | Nipa Laboratories | Preservative |
| Silicone emulsion (DC57134) 70% | 9:1 Blend of a 600 Pa.s PDMS and DC8566 aminosilicone fluid (TMN6 and CTAC emulsified) | Dow Corning | Hair conditioning agent |
| Perfume (100%) | | Givaudan | Perfume |
| Glydant (55%) | DMDM Hydantoin, 1,3-Bis(hydroxymethyl)-5,5-dimethylimidazolidine-2,4-dione | Lonza | Preservative |
| Disodium EDTA (100%) | Disodium salt of ethylene diamine tetra acetic acid. | Surfachem | Metal ion sequestrant |
| Kathon (1.5% active thiazolins) | 5-Chlor-2-methyl-4-isothiazolin-3-one and 2-methyl-4-isothiazolin-3-one mixture | Rohm & Haas | Preservative |
| Hallstar PEG 6000 DS C (100%) | PEG 150 DS, mix of di and mono stearate esters of high molecular weight PEG. | The Hallstar Co | Thickening agent, rheology modifier. |
| Oils mixture | 60% coconut oil, fully refined 35% mineral oil, M40 5% almond oil | Cargill FUCHS RITA | Hair conditioning agent |

**Table 2 Formulations of structured liquid compositions.**

| *Ingredient* | *U100* | *B100* | *F100* |
|---|---|---|---|
| Genamin BTLF | 1.00 | 2.85 | - |
| Lexamine S-13 | 1.00 | | 1.35 |
| Lanette | 4.00 | 4.00 | 3.00 |
| Purac | 0.32 | | 0.42 |
| Potassium chloride | 0.10 | | 0.30 |
| Nipagin | | 0.20 | |
| Silicone emulsion (DC57134) | 3.57 | 3.57 | - |
| Perfume | 0.60 | 0.60 | 0.50 |
| Glydant | 0.10 | - | 0.10 |
| Disodium EDTA | 0.10 | - | 0.10 |
| Kathon | 0.04 | - | 0.06 |
| Hallstar PEG 6000 DS C | - | - | 0.01 |
| Oils mixture | 1.50 | - | - |
| Water | 87.67 | 88.78 | 94.16 |

### Characterisation of viscosities

The viscosities for structured liquids were determined using a Brookfield RV viscometer (ex Brookfield Engineering Laboratories, Inc., Middleboro, MA, USA), fitted with a T-Bar B spindle and operated at a rotational speed of 0.5 rpm, and at room temperature between of 15 and 25°C, typically 20°C. The shear rate is lower than 1 s⁻¹. Whenever viscosity is mentioned in here, the dynamic viscosity (in mPa·s or Pa·s) is meant.

### Example 1 - Preparation of structured liquids using CDDM

A B100 structured liquid composition as defined in Table 2 was prepared by melting the fatty alcohol at a temperature of about 75°C in a batch mixer fitted with a re-circulation loop incorporating a Silverson high shear mixing device (model 275/400 supplied by Silverson Machines Ltd., Chesham, Buckinghamshire, UK) operating at a tip speed of 11 metres per second, and to which was added a mixture of cationic surfactant and water to form a dispersion at a temperature of about 65°C.The remaining ingredients as listed in Table 2 were added to the mixture after it had cooled down to a temperature of about 45°C, and were then gently mixed into the composition.

The product thus formed had a starting viscosity of 174,000 mPa·s (174 Pa·s) and was passed through the CDDM mixing device at a temperature of 20 to 25°C at flowrates varying between 75 and 300 L/h, and at pressures varying up to 46 bar, and at rotational speeds varying between 0 and 10,000 rpm (static and dynamic operation).

The viscosities of the compositions after the passage through the CDDM were determined, and are recorded in Table 3 as function of flowrate and rotor speed. The corresponding pressure drops are also recorded. The viscosities were determined 3 days after production of the samples, in order to equilibrate the samples.

**Table 3 Viscosity (in mPa·s) and pressure drop (in bar, between brackets []) as function of flowrate (in L/h) and rotor speed of the CDDM (in rpm); composition B100, starting viscosityof 174,000 mPa·s.**

| *Flowrate into CDDM [L*/*h]* | *Rotor speed of CDDM [rpm]* | | | |
|---|---|---|---|---|
| | *0* | *2,500* | *5,000* | *10,000* |
| 75 | 275,000 | 380,000 | 454,000 | 548,000 |
| | [24.6] | [n/a] | [17.4] | [12.7] |
| 150 | 408,000 | 370,000 | 399,000 | 458,000 |
| | [31.8] | [27.1] | [19.82] | [17.6] |
| 300 | 410,000 | 387,000 | 455,000 | 470,000 |
| | [31.8] | [36.6] | [31.7] | [29.4] |

The table above indicates that the viscosity of the B100 composition increases significantly on passage through the CDDM mixing device, under all operating conditions and can be increased by a factor in excess of 3. Further that viscosity can be achieved at a significantly lower pressure drop as the rotor speed increases.

### Example 2 - Preparation of structured liquids using CDDM

A composition of F100 structured liquid as defined in Table 2 was prepared similarly as in example 1. This mixture had a starting viscosity of 202,000 mPa·s (202 Pa·s) and was passed through the CDDM mixing device at a temperature of 20 to 25°C at at flowrates varying between 75 and 300 L/h, and at pressures varying up to 36 bar, and at rotational speeds varying between 0 and 10,000 rpm (static and dynamic operation). The viscosities of the compostions after the passage through the CDDM were determined, and are recorded in Table 4 as functions of flowrate and rotor speed. The corresponding pressure drops are also recorded. The viscosities were determined 3 days after production of the samples, in order to equilibrate the samples.

**Table 4 Viscosity (in mPa·s) and pressure drop (in bar, between brackets []) as function of flowrate (in Uh) and rotor speed of the CDDM (in rpm); composition F100, starting viscosity of 202,000 mPa·s.**

| *Flowrate into CDDM [L*/*h]* | *Rotor speed of CDDM [rpm]* | | | | |
|---|---|---|---|---|---|
| | *0* | *2,500* | *5,000* | *7,500* | *10,000* |
| 75 | 378,000 | 387,000 | 411,000 | 436,000 | 427,000 |
| | [21.9] | [12.6] | [12.4] | [12.6] | [12.6] |
| 150 | 442,000 | 396,000 | 423,000 | 417,000 | 418,000 |
| | [26.9] | [17.4] | [17.2] | [17.2] | [17.2] |
| 300 | 431,000 | 415,000 | 411,000 | 433,000 | 409,000 |
| | [36.3] | [26.7] | [24.5] | [26.7] | [24.4] |

The table above indicates that the viscosity of the F100 composition increases significantly under all operating conditions, and can be increased by a factor in excess of 2. Further the high viscosities can be achieved at a lower pressure drop when the rotor is not static.

### Example 3 - Preparation of structured liquids using CDDM (static operation)

A composition of U100 structured liquid as defined in Table 2 was prepared similarly as in example 1. The U100 structured liquid feedstock had a starting viscosity of 200,000 mPa·s (200 Pa·s). One fraction of that feedstock was then passed up to four times through the CDDM mixing device at 20-25°C, at a flowrate of 300 L/h, and under static conditions (0 rpm). Other portions of the composition (at 20°C) were diluted to varying degrees with water (at 20°C, with a hand held mixer until visually homogeneous) and then passed up to three times through the CDDM mixing device at 20-25°C and a flowrate of 300 L/h.

The batches of U100 composition were diluted to a content of 75%, 50%, or 37.5 wt% of U100, by gently mixing water into the composition U100, to give the compositions U75, U50, and U37.5, respectively. These liquids contain 75%, 50%, and 37.5% respectively of the raw materials of liquid U100.

**Table 5 Formulations of structured liquid U100 and diluted compositions.**

| *Ingredient* | *U100* | *U75* | *U50* | *U37.5* |
|---|---|---|---|---|
| Genamin BTLF | 1.00 | 0.75 | 0.50 | 0.38 |
| Lexamine S-13 | 1.00 | 0.75 | 0.50 | 0.38 |
| Lanette | 4.00 | 3.00 | 2.00 | 1.50 |
| Purac | 0.32 | 0.24 | 0.16 | 0.12 |
| Potassium chloride | 0.10 | 0.08 | 0.05 | 0.04 |
| Silicone emulsion (DC57134) | 3.57 | 2.68 | 1.79 | 1.34 |
| Perfume | 0.60 | 0.45 | 0.30 | 0.23 |
| Glydant | 0.10 | 0.08 | 0.05 | 0.04 |
| Disodium EDTA | 0.10 | 0.08 | 0.05 | 0.04 |
| Kathon | 0.04 | 0.03 | 0.02 | 0.02 |
| Oils mixture | 1.50 | 1.13 | 0.75 | 0.56 |
| Water | 87.67 | 90.75 | 93.84 | 95.38 |

The viscosities before any and after each such passage were determined and are recorded in Table 6 below as function of composition and number of passes. The corresponding pressure drops are also recorded. The viscosities were determined 3 days after production of the samples, in order to equilibrate the samples.

**Table 6 Viscosity (in mPa·s) and pressure drop (in bar, between brackets []) as function of dilution of composition U100 (percentage U100 at 100%, 75%, 50%, 37.5%, respectively, flowrate 300 Uh) and rotor speed 0 rpm (static CDDM mixer).**

| *Composition* | *Number of Passes* | | | |
|---|---|---|---|---|
| | *0* | *1* | *2* | *3* |
| U100 | 200,000 | 375,000 | 545,000 | 540,000 |
| | [-] | [53.6] | [53.6] | [48.6] |
| U75 | 190,000 | 396,000 | 454,000 | 397,000 |
| | [-] | [34.1] | [31.8] | [31.8] |
| U50 | 160,000 | 260,000 | 297,000 | 295,000 |
| | [-] | [29.4] | [29.5] | [27.1] |
| U37.5 | 56,000 | 185,000 | 182,000 | 204,000 |
| | [-] | [24.7] | [27.1] | [22.2] |

The viscosity at the number of passes of 0, is the starting viscoscity of the composition without the composition being passed through the mixing device. The table above indicates that the viscosities of each of the compositions increases significantly on passage through the CDDM mixing device, and can be increased on a second passage by overall factors with respect to the original 100 wt% U100 composition of about 2.75, about 2.25, and about 1.5 in the case of the 100, 75 and 50 wt% U100 compositions, respectively. Further, following a third passage, the viscosity of the 37.5 wt% U composition approximates to that of the original 100 wt% U100 composition, while the concentration of actives is much lower (only 37.5% of the actives).

The pressure drops across the CDDM mixing apparatus are dependent upon the viscosities and flowrates of the emerging structured liquids. In general the pressure drop is higher when the rotor speed is zero, meaning the CDDM is run in static mode, as compared to a situation that the rotor speed is larger than zero.

This shows that the structured liquids of the invention can be produced using both the rotating as well as the static operation.

### Conclusion

Examples 1 to 3 show that the CDMM mixing apparatus can affect the viscosity of the compositions dramatically. A well characterised extensional shear flow can produce structured liquids of greatly increased viscosity, also in case of significant dilution of the original formulation. And this is done at a low temperature below 35 to 40°C. A significantly increased water fraction with respect to the original composition can lead to considerable saving of raw materials. Viscosities up to 500,000 mPa·s can be achieved, with a good reproducibility. These high viscosities may be reduced by decreasing the amount of raw materials, therewith saving on the amount of raw materials needed.

## Claims

1. A method for production of a structured liquid composition comprising water, a fatty compound, and a cationic surfactant, comprising the step:
a) melting the fatty compound and mixing it with a mixture of cationic surfactant and water, or melting fatty compound and mixing it with the cationic surfactant, and mixing this mixture with water;
**characterised in that** in a next step
b) the mixture from step a) is introduced into a distributive and dispersive mixing apparatus of the Controlled Deformation Dynamic Mixer type,
wherein the mixer is suitable for inducing extensional flow in a liquid composition,
and wherein the mixer comprises closely spaced relatively moveable confronting surfaces at least one having a series of cavities therein in which the cavities on each surface are arranged such that, in use, the cross-sectional area for flow of the liquid successively increases and decreases by a factor of at least 5 through the apparatus.

2. A method according to claim 1, wherein after step b) further water is added to the mixture obtained from step b).

3. A method according to claim 1 or 2, wherein the cationic surfactant comprises a quaternary ammonium moiety.

4. A method according to any of claims 1 to 2, wherein the fatty compound comprises a fatty alcohol, preferably a C₁₆-C₂₂ fatty alcohol.

5. A method according to claim 4, wherein the weight ratio of cationic surfactant to fatty compound ranges from 1:1 to 1:10, preferably from 1:1.5 to 1:8, preferably from 1:2 to 1:5.

6. A method according to any of claims 1 to 5, wherein a silicone conditioning agent is added to the mixture from step a) prior to introducing the mixture into step b), at a concentration ranging from 0.01 % to 10% by weight of the total composition.

7. A method according to any of claims 1 to 6, wherein the mixture from step a) is introduced into the mixing apparatus at a temperature ranging from 10 to 40°C, preferably from 20 to 35°C.

8. A method according to any of claims 1 to 7, wherein the Controlled Deformation Dynamic Mixer comprises two confronting surfaces (1, 2), spaced by a distance (7), wherein the first surface (1) contains at least three cavities (3), wherein at least one of the cavities has a depth (9) relative to the surface (1),
wherein the second surface (2) contains at least three cavities (4) wherein at least one of the cavities has a depth (10) relative to the surface (2),
wherein the cross-sectional area for flow of the liquid available during passage through the apparatus successively increases and decreases at least 3 times, and
wherein the surface (1) has a length (5) between two cavities, and
wherein the surface (2) has a length (6) between two cavities, and
wherein the surfaces (1, 2) are positioned such that the corresponding lengths (5, 6) overlap to create a slit having an offset distance (8) or do not overlap creating a offset distance (81),
wherein the cavities are arranged such that the cross-sectional area for flow of the liquid available during passage through the apparatus successively increases in the cavities and decreases in the slits by a factor of at least 5 and
wherein the distance (7) between the two surfaces (1,2) is between 2 micrometer and 300 micrometer, and wherein
either the ratio between the offset distance (8) and the distance (7) between the two surfaces (1, 2) ranges from 0 to 250,
or wherein the ratio between the offset distance (81) and the distance (7) between the two surfaces (1, 2) ranges from 0 to 30.

9. A method according to any of claims 1 to 8, wherein the mixing apparatus is operated at a pressure less than 200 bar.

10. A method according to any of claims 1 to 9, wherein one of the surfaces rotates relative to the other surface at a frequency between 1,000 and 25,000 rotations per minute, preferably between 3,000 and 12,000 rotations per minute.

11. A method according to any of claims 1 to 9, wherein the surfaces are static with respect to each other.

12. A structured liquid obtained by the method according to any one of claims 1 to 11, containing fatty compound at maximally 3% by weight, and cationic surfactant at maximally 2% by weight, and water, and wherein the structured liquid has a dynamic viscosity of at least 190,000 mPa·s, preferably at least 220,000 mPa.s, measured at a shear rate lower than 0.5 s⁻¹ and a temperature of 20°C.

13. A structured liquid according to claim 12, having a dynamic viscosity of at least 190,000 mPa·s, preferably at least 220,000 mPa.s, measured using a Brookfield RV viscometer, fitted with a T-Bar B spindle and operated at a rotational speed of 0.5 rpm, and at a temperature of 20°C.

14. A structured liquid according to claim 12 or 13, wherein the fatty compound comprises a fatty alcohol, preferably a C₁₆-C₂₂ fatty alcohol.

15. Use of a structured liquid according to any of claims 12 to 14 or prepared according to the method of any of claims 1 to 11 as hair conditioner.

## Patentansprüche

1. Verfahren zur Herstellung einer strukturierten flüssigen Zusammensetzung, umfassend Wasser, eine Fettverbindung und ein kationisches Tensid, umfassend den Schritt:
a) Schmelzen der Fettverbindung und deren Mischen mit einer Mischung von kationischem Tensid und Wasser, oder Schmelzen der Fettverbindung und deren Mischen mit dem kationischem Tensid und Mischen dieser Mischung mit Wasser;
**dadurch gekennzeichnet, dass** in einem nächsten Schritt
b) die Mischung von Schritt a) in eine distributive und dispersive Mischvorrichtung vom Typ des dynamischen Mischers mit gesteuerter Verformung eingebracht wird,
wobei der Mischer dazu geeignet ist, in einer flüssigen Zusammensetzung Dehnströmung hervorzurufen,
und wobei der Mischer eng beabstandete, relativ zueinander bewegliche, gegeneinander anlaufende Flächen umfasst, wobei mindestens eine davon eine Reihe von Löchern darin hat, wobei die Löcher auf jeder Fläche derart angeordnet sind, dass im Betrieb die Querschnittsfläche für die Strömung der Flüssigkeit durch die Vorrichtung nacheinander um einen Faktor von mindestens 5 zunimmt und abnimmt.

2. Verfahren gemäß Anspruch 1, wobei nach Schritt b) der aus Schritt b) erhaltenen Mischung weiteres Wasser zugesetzt wird.

3. Verfahren gemäß Anspruch 1 oder 2, wobei das kationische Tensid einen quaternären Ammoniumrest aufweist.

4. Verfahren gemäß irgendeinem der Ansprüche 1 bis 2, wobei die Fettverbindung einen Fettalkohol umfasst, vorzugsweise einen C₁₈-C₂₂ Fettalkohol.

5. Verfahren gemäß Anspruch 4, wobei das Gewichtsverhältnis von kationischem Tensid zu Fettverbindung im Bereich von 1:1 bis 1:10 liegt, vorzugsweise von 1:1,5 bis 1:8, vorzugsweise von 1:2 bis 1:5.

6. Verfahren gemäß irgendeinem der Ansprüche 1 bis 5, wobei der Mischung aus dem Schritt a) vor dem Einbringen der Mischung in den Schritt b) ein Silicon-Konditioniermittel mit einer Konzentration im Bereich von 0,01 Gew.-% bis 10 Gew.-% der gesamten Zusammensetzung zugesetzt wird.

7. Verfahren gemäß irgendeinem der Ansprüche 1 bis 6, wobei die Mischung aus Schritt a) bei einer Temperatur im Bereich von 10 bis 40°C, vorzugsweise von 20 bis 35°C in die Mischvorrichtung eingebracht wird.

8. Verfahren gemäß irgendeinem der Ansprüche 1 bis 7, wobei der dynamische Mischer mit gesteuerter Verformung zwei gegeneinander anlaufende Flächen (1, 2) umfasst, die in einem Abstand (7) beabstandet sind,
wobei die erste Fläche (1) mindestens 3 Löcher (3) umfasst, wobei mindestens eines der Löcher eine Tiefe (9) relativ zu der Fläche (1) aufweist, wobei die zweite Fläche (2) mindestens 3 Löcher (4) umfasst, wobei mindestens eines der Löcher eine Tiefe (10) relativ zu der Fläche (4) aufweist, wobei die für die Strömung der Flüssigkeit durch die Vorrichtung während des Durchgangs durch die Vorrichtung zur Verfügung stehende Querschnittsfläche mindestens 3 mal nacheinander zunimmt und abnimmt, und
wobei die Fläche (1) eine Länge (5) zwischen zwei Löchern aufweist,
wobei die Fläche (2) eine Länge (6) zwischen zwei Löchern aufweist und
wobei die Flächen (1, 2) so positioniert sind, dass die entsprechenden Längen (5, 6) sich überlappen, um einen Schlitz mit einem Versatzabstand (8) zu erzeugen, oder sich nicht überlappen um einen Versatzabstand (81) zu erzeugen,
wobei die Löcher so angeordnet sind, dass die für die Strömung der Flüssigkeit durch die Vorrichtung während des Durchgangs durch die Vorrichtung zur Verfügung stehende Querschnittsfläche um einen Faktor von mindestens 5 nacheinander in den Löchern zunimmt und in den Schlitzen abnimmt und
wobei der Abstand (7) zwischen den beiden Flächen (1, 2) zwischen 2 Mikrometern und 300 Mikrometern beträgt und wobei
entweder das Verhältnis zwischen dem Versatzabstand (8) und dem Abstand (7) zwischen den beiden Flächen (1, 2) im Bereich von 0 bis 250 liegt,
oder wobei das Verhältnis zwischen dem Versatzabstand (81) und dem Abstand (7) zwischen den beiden Flächen (1, 2) im Bereich von 0 bis 30 liegt.

9. Verfahren gemäß irgendeinem der Ansprüche 1 bis 8, wobei die Mischvorrichtung bei einem Druck von weniger als 200 bar betrieben wird.

10. Verfahren gemäß irgendeinem der Ansprüche 1 bis 9, wobei eine der Flächen relativ zu der anderen Fläche mit einer Frequenz zwischen 1.000 und 25.000 Umdrehungen pro Minute rotiert, vorzugsweise zwischen 3.000 und 12.000 Umdrehungen pro Minute.

11. Verfahren gemäß irgendeinem der Ansprüche 1 bis 9, wobei die Flächen in Bezug zueinander statisch sind.

12. Strukturierte Flüssigkeit, erhalten mittels des Verfahrens gemäß irgendeinem der Ansprüche 1 bis 11, enthalten Fettverbindung mit maximal 3 Gew.-% und kationisches Tensid mit maximal 2 Gew.-% und Wasser, und wobei die strukturierte Flüssigkeit eine dynamische Viskosität von mindestens 190.000 mPa·s, vorzugsweise mindestens 220.000 mPa·s aufweist, gemessen bei einer Scherungsgeschwindigkeit niedriger als 0,5 s⁻¹ und bei einer Temperatur von 20°C.

13. Strukturierte Flüssigkeit gemäß Anspruch 12, mit einer dynamischen Viskosität von mindestens 190.000 mPa·s, vorzugsweise mindestens 220.000 mPa·s gemessen mit einem Brookfield RV Viskosimeter, das mit einer T-Bar B Spindel ausgerüstet ist und mit einer Umdrehungsgeschwindigkeit von 0,5 Umdrehungen pro Minute und bei einer Temperatur von 20°C betrieben wird.

14. Strukturierte Flüssigkeit gemäß Anspruch 12 oder 13, wobei die Fettverbindung einen Fettalkohol umfasst, vorzugsweise einen C₁₈-C₂₂ Fettalkohol.

15. Verwendung einer strukturierten Flüssigkeit gemäß irgendeinem der Ansprüche 12 bis 14 oder hergestellt mit dem Verfahren gemäß irgendeinem der Ansprüche 1 bis 11 als Haarspülung.

## Revendications

1. Procédé pour produire une composition liquide structurée comprenant de l'eau, un composé gras, et un tensioactif cationique, comprenant l'étape de :
a) fusion du composé gras et mélange de celui-ci avec un mélange de tensioactif cationique et d'eau, ou fusion du composé gras et mélange de celui-ci avec le tensioactif cationique, et mélange de ce mélange avec de l'eau ;
**caractérisé en ce que**, dans une étape suivante,
b) le mélange obtenu dans l'étape a) est introduit dans un dispositif de mélange distributif et dispersif du type mélangeur dynamique à déformation contrôlée,
dans lequel le mélangeur est adapté pour induire un écoulement extensionnel dans une composition liquide,
et dans lequel le mélangeur comprend des surfaces en confrontation relativement mobiles et étroitement espacées, dont au moins une a une série de cavités dans celle-ci, les cavités sur chaque surface étant agencées de façon que, lors de l'utilisation, la surface en coupe transversale pour l'écoulement du liquide augmente et diminue successivement d'un facteur d'au moins 5 à travers le dispositif.

2. Procédé selon la revendication 1, dans lequel, après l'étape b), davantage d'eau est ajoutée au mélange obtenu dans l'étape b).

3. Procédé selon la revendication 1 ou 2, dans lequel le tensioactif cationique comprend un fragment ammonium quaternaire.

4. Procédé selon l'une quelconque des revendications 1 et 2, dans lequel le composé gras comprend un alcool gras, de préférence un alcool gras en C₁₆ à C₂₂.

5. Procédé selon la revendication 4, dans lequel le rapport en poids du tensioactif cationique au composé gras est situé dans la plage allant de 1/1 à 1/10, de préférence de 1/1,5 à 1/8, de préférence de 1/2 à 1/5.

6. Procédé selon l'une quelconque des revendications 1 à 5, dans lequel un agent de conditionnement siliconé est ajouté au mélange obtenu dans l'étape a) avant introduction du mélange dans l'étape b), à une concentration située dans la plage allant de 0,01 % à 10 % en poids de la composition totale.

7. Procédé selon l'une quelconque des revendications 1 à 6, dans lequel le mélange obtenu dans l'étape a) est introduit dans le dispositif de mélange à une température située dans la plage allant de 10 à 40°C, de préférence de 20 à 35°C.

8. Procédé selon l'une quelconque des revendications 1 à 7, dans lequel le mélangeur dynamique à déformation contrôlée comprend deux surfaces en confrontation (1, 2) espacées par une distance (7),
dans lequel la première surface (1) contient au moins trois cavités (3), au moins l'une des cavités ayant une profondeur (9) par rapport à la surface (1),
dans lequel la deuxième surface (2) contient au moins trois cavités (4), au moins l'une des cavités ayant une profondeur (10) par rapport à la surface (2),
dans lequel la surface en coupe transversale pour l'écoulement du liquide disponible durant le passage à travers le dispositif augmente et diminue successivement au moins 3 fois, et
dans lequel la surface (1) a une longueur (5) entre deux cavités, et
dans lequel la surface (2) a une longueur (6) entre deux cavités, et
dans lequel les surfaces (1, 2) sont positionnées de façon que les longueurs correspondantes (5, 6) se chevauchent pour créer une fente ayant une distance de déport (8) ou ne se chevauchent pas en créant une distance de déport (81),
dans lequel les cavités sont agencées de façon que la surface en coupe transversale pour l'écoulement du liquide disponible durant le passage à travers le dispositif augmente dans les cavités et diminue dans les fentes successivement par un facteur d'au moins 5, et
dans lequel la distance (7) entre les deux surfaces (1, 2) est comprise entre 2 micromètres et 300 micromètres, et dans lequel
soit le rapport entre la distance de déport (8) et la distance (7) entre les deux surfaces (1, 2) est situé dans la plage allant de 0 à 250,
soit le rapport entre la distance de déport (81) et la distance (7) entre les deux surfaces (1, 2) est situé dans la plage allant de 0 à 30.

9. Procédé selon l'une quelconque des revendications 1 à 8, dans lequel le dispositif de mélange fonctionne sous une pression inférieure à 200 bar.

10. Procédé selon l'une quelconque des revendications 1 à 9, dans lequel l'une des surfaces tourne par rapport à l'autre surface à une fréquence comprise entre 1 000 et 25 000 tours par minute, de préférence entre 3 000 et 12 000 tours par minute.

11. Procédé selon l'une quelconque des revendications 1 à 9, dans lequel les surfaces sont statiques l'une par rapport à l'autre.

12. Liquide structuré obtenu par le procédé selon l'une quelconque des revendications 1 à 11, contenant un composé gras à raison de 3 % en poids au maximum, et un tensioactif cationique à raison de 2 % en poids au maximum, et de l'eau, lequel liquide structuré a une viscosité dynamique d'au moins 190 000 mPa.s, de préférence d'au moins 220 000 mPa.s, mesurée à une vitesse de cisaillement inférieure à 0,5 s⁻¹ et à une température de 20°C.

13. Liquide structuré selon la revendication 12, ayant une viscosité dynamique d'au moins 190 000 mPa.s, de préférence d'au moins 220 000 mPa.s, mesurée par utilisation d'un viscosimètre Brookfield RV muni d'une broche B à barre en T et fonctionnant à une vitesse de rotation de 0,5 t/min et à une température de 20°C.

14. Liquide structuré selon la revendication 12 ou 13, dans lequel le composé gras comprend un alcool gras, de préférence un alcool gras en C₁₆ à C₂₂.

15. Utilisation d'un liquide structuré selon l'une quelconque des revendications 12 à 14 ou préparé conformément au procédé de l'une quelconque des revendications 1 à 11, en tant que conditionneur capillaire.
